# FASCICULE DE BREVET EUROPEEN

(11) **EP 0 976 412 B1**
(45) Date de publication et mention de la délivrance du brevet: **11.02.2004**
(21) Numéro de dépôt: 99401878.6
(22) Date de dépôt: 23.07.1999
(51) Int. Cl.: A61L 26/00

(54) **Dispositif de traitement de plaies et procédé pour sa fabrication**
Wundverband und Verfahren zu dessen Herstellung
Device for wound treatment and method of producing the same

(30) Priorité: 31.07.1998 FR 9809858
(43) Date de publication de la demande: 02.02.2000
(73) Titulaire: Les Laboratories Brothier, 92000 Nanterre (FR)
(72) Inventeur: Maingault, Philippe, 92000 Nanterre (FR); Dellacherie, Edit, 54220 Malzeville (FR); Hubert, Patrick, 54000 Nancy (FR); Houzelle, Marie-Christine, 57640 Vigy (FR); Pelletier, Sophie, 88350 Liffol le Grand (FR)
(74) Mandataire: Bloch, Gérard

(56) Documents cités:
- EP-A- 0 386 960
- EP-A- 0 724 888

## Description

L'invention concerne un produit, ou dispositif, de traitement destiné à soigner des plaques de nécrose, des plaies aiguës de type chirurgical et autres types de plaies.

Seront considérés ici principalement deux types de produits de traitement de plaies.

Les produits du premier type sont naturels et comprennent des molécules de polysaccharide. Il s'agit par exemple de produits à base d'alginate. Les produits de ce type ont des propriétés intrinsèques, naturelles, très efficaces pour le traitement des plaies.

Les produits du second type sont synthétiques et, de ce fait, ont des propriétés curatives moins performantes.

Les produits de traitement, qu'ils soient naturels ou synthétiques, s'utilisent généralement soit à l'état de solution soit à l'état de gel.

A l'état de solution, le produit de traitement s'applique et se répand facilement sur la plaie avec laquelle il entre en contact intime. Cependant, la répartition de la solution n'est généralement pas uniforme et la solution s'écoule inévitablement en dehors de la plaie, même avec un pansement de protection et d'étanchéité.

A l'état de gel, le produit de traitement peut certes être plus facilement maintenu contre la plaie à l'aide d'un pansement, mais n'entre pas en contact intime avec elle.

On connaît également des gels de traitement synthétiques, réversibles. Ces gels sont dits "réversibles" du fait qu'ils peuvent changer d'état, de façon réversible, en passant de l'état de gel à l'état de solution et inversement. Cette réversibilité gel/solution est due aux macromolécules synthétiques constitutives du produit, aux conformations intrinsèquement réversibles.

Une telle réversibilité présente un grand intérêt pour le traitement des plaies. En effet, elle permet notamment d'appliquer le produit de traitement à l'état liquide sur une plaie, de façon à ce qu'il se répande sur la plaie, l'épouse et établisse un contact intime avec elle. Après un temps de prise, le produit se gélifie en adhérant à la plaie, sans écoulement.

En outre, le gel situé à proximité de la plaie peut se liquéfier, sous l'effet de la température notamment. Grâce à cela, le produit reste toujours en contact intime avec la plaie, même en cas de mouvements du patient ou d'évolution de la plaie.

L'invention vise à offrir un produit de traitement réversible présentant des propriétés encore meilleures que celles des produits de traitement réversibles connus.

A cet effet, l'invention concerne un dispositif naturel de traitement de plaie, agencé pour changer d'état de façon réversible, par passage de l'état de gel à l'état de solution et inversement, comprenant des macromolécules de polysaccharide et des chaînes aliphatiques fixées sur une seule macromolécule de polysaccharide.

Le dispositif de traitement de l'invention non seulement est réversible mais possède également, intrinsèquement, des propriétés naturelles curatives très efficaces.

En outre, le dispositif de traitement possède les propriétés intrinsèques aux chaînes aliphatiques, particulièrement intéressantes pour le traitement des plaies, et variant en fonction de la longueur de la chaîne aliphatique.

A titre d'exemple, les produits de traitement comprenant des chaînes aliphatiques à huit atomes de carbone ont la propriété d'inhiber la division de micro-organismes par "perméation membranaire", selon le vocabulaire utilisé par l'homme du métier.

Les produits de traitement comprenant des chaînes aliphatiques à douze atomes de carbone sont indiqués pour le traitement de plaies chroniques en phase de détersion, par exemple des plaques de nécrose.

Les produits de traitement comprenant des chaînes aliphatiques à vingt atomes de carbone sont indiqués pour le traitement de plaies chroniques en phase d'épidermisation.

Pour une plaie aiguë de type chirurgical par exemple, les produits de traitement comprenant des chaînes aliphatiques à seize atomes de carbone et/ou des chaînes aliphatiques à douze atomes de carbone sont particulièrement indiqués car ils ont des propriétés biologiques et de bioadhésivité permettant de réduire les phénomènes d'adhérence tissulaire aux interfaces.

On peut bien sûr fixer des chaînes aliphatiques de longueurs différentes sur des macromolécules de polysaccharide, afin d'obtenir un produit présentant de multiples propriétés, et accroître encore l'efficacité du produit.

On notera ici que le document EP-0 341 745 enseigne un produit obtenu par réticulation chimique, comprenant des macromolécules de polysaccharide reliées les unes aux autres par des liaisons transversales, ou "ponts de réticulation", consistant en des chaînes aliphatiques. Chacune de ces chaînes aliphatiques relie deux macromolécules de polysaccharide par des liaisons chimiques de forte énergie. Il en résulte que le produit est à l'état solide, ou à l'état de gel, et irréversible.

Avantageusement, les chaînes aliphatiques sont fixées par une liaison chimique.

Ladite liaison chimique peut être une liaison ionique ou une liaison covalente, par exemple une liaison ester.

Avantageusement encore, les chaînes aliphatiques fixées sur une macromolécule de polysaccharide sont, à l'état de gel, associées à des chaînes aliphatiques fixées sur au moins une autre macromolécule de polysaccharide.

Les chaînes aliphatiques constituent ainsi des bras hydrocarbonés par le biais desquels les macromolécules s'associent.

L'invention concerne en outre un procédé de fabrication du dispositif de traitement de plaie ci-dessus, au cours duquel, on prend des macromolécules de polysaccharide et des chaînes aliphatiques pourvues chacune d'un unique groupe de liaison et on met en contact lesdites macromolécules de polysaccharide et lesdites chaînes aliphatiques afin de fixer des chaînes aliphatiques sur les macromolécules.

L'invention concerne encore un dispositif naturel de traitement de plaie tel que celui défini ci-dessus, dans lequel des molécules d'un principe actif sont piégées dans des alvéoles du dispositif à l'état de gel.

L'invention concerne enfin un dispositif naturel de traitement de plaie tel que celui défini ci-dessus, dans lequel des cellules vivantes sont piégées dans des alvéoles du dispositif à l'état de gel.

L'invention sera mieux comprise à l'aide de la description suivante de différentes formes de réalisation du dispositif de traitement de plaies et de différents modes de réalisation du procédé de fabrication du dispositif de traitement, en référence au dessin annexé sur lequel:
- la figure 1 représente trois macromolécules du dispositif de traitement à l'état de gel, selon une forme de réalisation particulière;
- la figure 2 représente des étapes du procédé de fabrication du dispositif de traitement selon l'un des modes de réalisation;
- la figure 3 représente le dispositif de traitement dans lequel sont piégées des molécules d'un principe actif, avant la délivrance du principe actif;
- la figure 4 représente le dispositif de traitement de la figure 3 pendant la délivrance du principe actif;
- la figure 5 représente le dispositif de traitement dans lequel sont piégées des cellules vivantes, avant la délivrance de ces cellules, et
- la figure 6 représente le dispositif de traitement de la figure 5, pendant la délivrance des cellules.

Le dispositif, ou produit, de traitement de plaie comprend des macromolécules de polysaccharide 10, 20, 30, en l'espèce des macromolécules d'alginate, et des chaînes aliphatiques 11-15, 21-24, 31-33, ici de formule CₙH₂ₙ₊₁. Le nombre d'atomes de carbone des chaînes aliphatiques est ici égal à 8.

Dans l'exemple particulier de la description, chaque chaîne aliphatique 11-15, 21-24, 31-33 est pourvue d'un unique groupe de fixation, en l'espèce un groupe amine ionisé NH₃⁺, par l'intermédiaire duquel elle est fixée sur une seule macromolécule d'alginate 10, 20, 30, par une liaison chimique consistant en une liaison ionique entre le groupe amine ionisé NH₃⁺ et un groupe carboxylate ionisé COO⁻ de la macromolécule d'alginate.

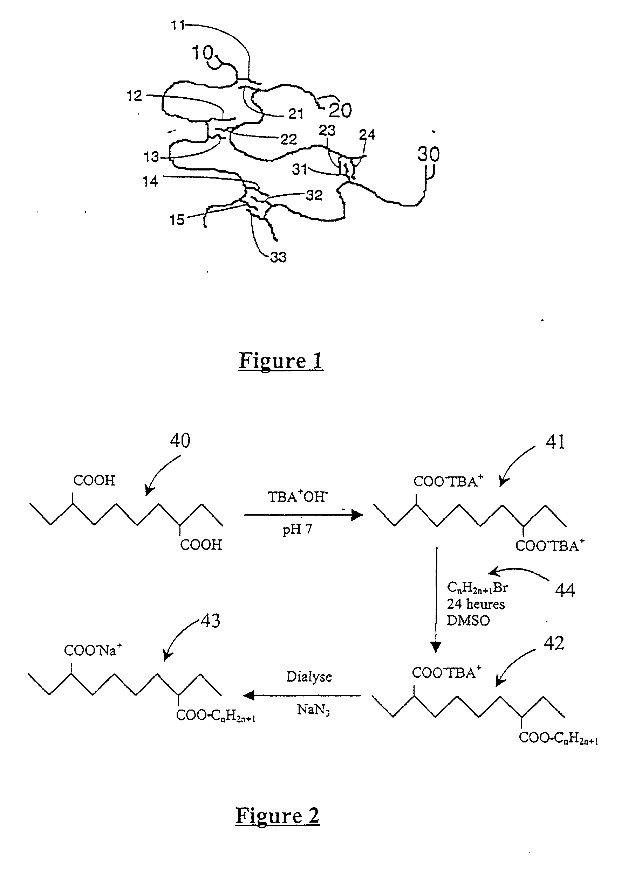

Les chaînes aliphatiques 11-1.5, 21-24, 31-33 de chaque macromolécule 10, 20, 30 forment des bras hydrocarbonés qui, à l'état de gel du dispositif, sont associés à des bras hydrocarbonés d'au moins une autre macromolécule voisine. Sur la figure 1, on peut voir que les bras hydrocarbonés de chacune des macromolécules 10 (20; 30) sont associés à des bras hydrocarbonés des deux autres macromolécules 20, 30 (10, 30; 10, 20). Chaque association de bras hydrocarbonés comprend un nombre m de bras hydrocarbonés, avec m entier naturel supérieur ou égal à deux. Les bras hydrocarbonés 11/21, 12/22/13, 14/32/15/33, 23/31/24 de chaque association de bras sont reliés entre eux par des liaisons physiques consistant en des interactions hydrophobes. Les macromolécules d'alginate 10, 20, 30 s'associent ainsi les unes avec les autres, par l'intermédiaire de leurs bras hydrocarbonés 11-15, 21-24, 31-3 3 .

Les interactions hydrophobes entre les bras associés 11/21, 12/22/13, 14/32/15/33, 23/31/24 des macromolécules 10, 20, 30 sont d'énergie à la fois suffisamment forte pour assurer une résistance suffisante au dispositif à l'état de gel, et suffisamment faible pour permettre à ce gel de passer à l'état de solution, sous l'effet de contraintes extérieures telles que la température, les contraintes mécaniques, le pH, la force ionique du milieu, etc. En outre, ce changement d'état est réversible. En d'autres termes, sous l'effet de contraintes extérieures, le dispositif peut passer de l'état de gel à l'état de solution, par séparation des bras hydrocarbonés associés 11/21, 12/22/13, 14/32/15/33, 23/31/24 et, inversement, de l'état de solution à l'état de gel par association des bras hydrocarbonés 11-15, 21-24, 31-33.

Grâce à cela, on peut notamment couler le dispositif d'alginate à l'état de solution sur la plaie à traiter, de façon à ce qu'il épouse la plaie et entre en contact intime avec elle. Après un temps de prise, le dispositif se gélifie et adhère alors intimement à la plaie, sans risque d'écoulement. Puis, sous l'effet de la force ionique du milieu biologique tissulaire et/ou du pH de celui-ci, le gel d'alginate situé à proximité de la plaie se liquéfie, de sorte qu'en dépit d'éventuelles contraintes mécaniques (évolution de la plaie, mouvements du patient), le contact intime entre le dispositif de traitement et la plaie sera toujours assuré.

On pourrait également fixer sur des macromolécules de polysaccharide des chaînes aliphatiques possédant un nombre d'atomes de carbone égal à six, sept ou supérieur à huit, afin d'obtenir un dispositif de traitement réversible.

En outre, un même dispositif de traitement de plaies pourrait comprendre des chaînes aliphatiques de longueurs diverses.

Dans une variante, les chaînes aliphatiques sont fixées par des liaisons covalentes, en l'espèce des liaisons ester, comme cela sera explicité plus loin.

Après la description du dispositif de traitement de l'invention, le procédé de fabrication de ce dispositif, suivant deux modes de réalisation, va maintenant être décrit. Ces deux modes de réalisation sont les suivants:
1) fixation de chaînes aliphatiques, par liaison covalente, sur des macromolécules d'alginate et
2) fixation de chaînes aliphatiques, par liaison ionique, sur des macromolécules d'alginate.

### 1) Fixation de chaînes aliphatiques, par liaison covalente, sur des macromolécules d'alginate

En référence à la figure 2, on prend une suspension aqueuse d'acide alginique à 2% en poids. La suspension comprend des macromolécules d'acide alginique 40 comportant chacune une pluralité de groupements fonctionnels carboxyliques. Ces groupements fonctionnels comprennent, pour la plupart, un groupe carboxylique COOH, pour une faible proportion d'entre eux, un groupe carboxylate COO⁻ relié à un ion sodium Na⁺.

On neutralise la suspension par de l'hydroxyde de tétrabutyl ammonium (TBA⁺OH⁻) de façon à ce que le pH soit égal à 7. Les groupements tétrabutyl ammonium TBA⁺ se substituent aux protons d'hydrogène H⁺ dans les groupements fonctionnels carboxyliques des macromolécules d'acide alginique 40. Les macromolécules de polysaccharide 41 ainsi obtenues sont celles d'un dérivé ammonium de l'alginate comprenant des groupements fonctionnels carboxyliques (COO⁻TBA⁺) très réactifs.

On place ensuite, pendant vingt quatre heures, les macromolécules 41 dans un milieu solvant, en l'espèce du diméthylsulfoxyde DMSO, en présence de molécules 44 d'un dérivé bromé aliphatique, de formule CₙH₂ₙ₊₁Br. Le nombre n d'atomes de carbones des molécules 44 est ici égal à douze. Chaque molécule 44 comprend une chaîne aliphatique (de formule CₙH₂ₙ₊₁) et un atome de brome (Br) constituant un unique groupe de fixation de la chaîne aliphatique. On met ainsi en contact les macromolécules de polysaccharide 41 et les chaînes aliphatiques 44.

Les molécules 44 du dérivé bromé aliphatique réagissent sur les macromolécules 41 du dérivé ammonium d'alginate. Des chaînes aliphatiques CₙH₂ₙ₊₁ se substituent aux groupes tétrabutylammonium TBA⁺ de certains des groupements fonctionnels des macromolécules 41 du dérivé d'alginate, en perdant leur atome de brome. Le taux de substitution est de l'ordre de 8%. On fixe ainsi des chaînes aliphatiques CₙH₂ₙ₊₁ sur les macromolécules d'alginate. Chacune de ces chaînes aliphatiques fixées est reliée à un groupe carboxylate (COO⁻) par une liaison covalente, en l'espèce une liaison ester. Les chaînes aliphatiques sont chacune fixées à une seule macromolécule d'alginate. On obtient les macromolécules 42 représentées sur la figure 2.

Enfin, on dialyse la solution en présence d'azide de sodium (NaN₃) afin de substituer les groupes tétrabutylammonium TBA⁺ restant des macromolécules 42 par des ions sodium Na⁺, en se débarrassant ainsi de la fonctionnalité ammonium.

On obtient le dispositif final d'alginate réversible, destiné à être utilisé pour le traitement de plaies, comprenant des macromolécules d'alginate 43, représentées sur la figure 2, comportant chacune environ 92% de groupements fonctionnels carboxylates de sodium (de formule COO⁻Na⁺) et 8% de groupements fonctionnels estérifiés par des chaînes aliphatiques à douze atomes de carbones (de formule COO-CₙH₂ₙ₊₁).

### 2) Fixation de chaînes aliphatiques, par liaison ionique, sur des macromolécules d'alginate

On prend deux grammes d'alginate de sodium que l'on met en suspension dans une solution hydroalcoolique, en l'espèce 140 millilitres d'éthanol à 90%.

On ajoute dans la solution des molécules d'amine aliphatique de formule CₙH₂ₙ₊₁NH₂. Le nombre n d'atomes de carbone des molécules d'amine aliphatique est ici égal à 12. Chaque molécule d'amine aliphatique comprend une chaîne aliphatique (de formule CₙH₂ₙ₊₁) et un unique groupe amine (de formule NH₂) constituant un groupe de fixation pour la chaîne aliphatique. En solution, les groupes amines des molécules d'amine aliphatique s'ionisent en captant un proton. On active ainsi les groupes de fixation des chaînes aliphatiques par ionisation. Chaque molécule d'amine aliphatique comprend alors un unique groupe de fixation actif, de formule NH₃⁺.

Dans la solution, les macromolécules d'alginate sont en contact avec les molécules d'amine aliphatique ionisées.

On agite la solution à température ambiante pendant 90 minutes. Pendant cette période, des molécules d'amine aliphatique ionisées (CₙH₂ₙ₊₁NH₃⁺) réagissent sur les macromolécules d'alginate, en se substituant aux protons de certains groupements fonctionnels carboxyliques des macromolécules d'alginate. On fixe ainsi des chaînes aliphatiques sur les macromolécules d'alginate, par l'intermédiaire de leur groupe de fixation actif NH₃⁺. Les chaînes aliphatiques fixées sont chacune reliées à une seule macromolécule d'alginate par l'intermédiaire d'une liaison ionique entre le groupe de fixation (NH₃⁺) et un groupe carboxylate (COO⁻) de la macromolécule d'alginate.

Les macromolécules d'alginate ainsi obtenues comprennent donc des groupes carboxylates (COO⁻) reliés, pour certains, à un atome de sodium (Na⁺) par liaison ionique, et, pour les autres, à une chaîne aliphatique (CₙH₂ₙ₊₁) par l'intermédiaire d'un groupe de fixation (NH₃⁺), par liaison ionique.

Puis on procède à plusieurs lavages dans une solution d'éthanol, puis dans une solution de dioxane et enfin dans une solution d'acétone, afin d'obtenir un alginate modifié sec qui fournira un gel d'alginate réversible, après dissolution dans de l'eau.

L'utilisation du dispositif de traitement décrit plus haut pour la délivrance d'un principe actif va maintenant être décrite.

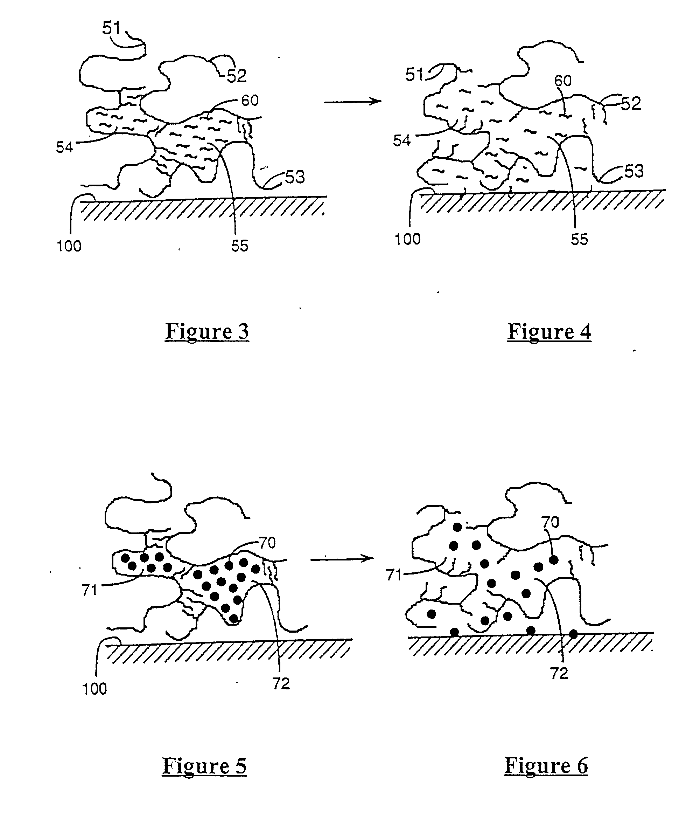

Dans le dispositif de traitement à l'état de gel (figure 3), les macromolécules d'alginate 51-53 sont associées entre elles, par l'intermédiaire de leurs bras hydrocarbonés associés entre eux. Du fait de ces associations entre macromolécules, la structure du gel, au niveau macromoléculaire, est alvéolaire, les parois des alvéoles étant constituées par des bras hydrocarbonés associés entre eux et par des portions de squelette de macromolécule d'alginate. Les alvéoles 54-55, ou réservoirs, du dispositif font fonction de pièges pour d'autres molécules.

Sur la figure 3, des molécules 60 d'un principe actif, destinées à être délivrées sur leur site d'application, autrement dit sur la plaie à traiter 100, sont piégées à l'intérieur des alvéoles 54-55.

Les molécules 60 du principe actif ont été introduites dans le dispositif de traitement à l'état de solution. Après un temps de prise, dans des conditions appropriées, la solution s'est gélifiée en piégeant les molécules 60 du principe actif dans les alvéoles 54-55 du gel.

Le gel de traitement contenant les molécules 60 du principe actif, appliqué sur une plaie, se liquéfie au voisinage immédiat de la peau, sous l'effet de contraintes extérieures telles que la température corporelle et les forces ioniques du milieu. Lors de la liquéfaction, les bras associés se séparent. Il en résulte que les alvéoles 54-55 s'ouvrent en libérant les molécules 60 du principe actif sur la plaie 100.

Dans une variante (figure 4), à la place du principe actif, on piège des cellules vivantes 70, animales ou végétales, dans les alvéoles 71, 72 du gel de traitement réversible. Il peut par exemple s'agir de cellules destinées à reconstituer des cartilages altérés.

Dans ce cas, l'ouverture des alvéoles 71, 72, pour la libération des cellules 70, pourrait être induite par des contraintes extérieures, analogues à celles explicitées plus haut (températures corporelles, forces ioniques, contraintes mécaniques, etc.) et/ou par des contraintes intérieures dues à une croissance des cellules piégées.

## Revendications

1. Dispositif naturel de traitement de plaie, agencé pour changer d'état de façon réversible, par passage de l'état de gel à l'état de solution et inversement, comprenant des macromolécules de polysaccharide (10, 20, 30) et des chaînes aliphatiques (11-15, 21-24, 31-33) fixées sur une seule macromolécule de polysaccharide.

2. Dispositif selon la revendication 1, dans lequel les chaînes aliphatiques (11-15, 21-24, 31-33) sont fixées par une liaison chimique.

3. Dispositif selon la revendication 2, dans lequel ladite liaison chimique est une liaison ionique.

4. Dispositif selon la revendication 2, dans lequel ladite liaison chimique est une liaison covalente.

5. Dispositif selon la revendication 4, dans lequel la liaison chimique est une liaison ester.

6. Dispositif selon l'une des revendications 1 à 5, dans lequel chaque chaîne aliphatique est fixée par l'intermédiaire d'un groupe de fixation.

7. Dispositif selon l'une des revendications 1 à 6, dans lequel les chaînes aliphatiques (21-24) fixées sur une macromolécule de polysaccharide (20) sont, à l'état de gel, associées avec des chaînes aliphatiques (11, 12/13, 31) fixées sur au moins une autre macromolécule de polysaccharide (10, 30).

8. Dispositif selon la revendication 7, dans lequel les chaînes aliphatiques associées (11/21, 12/22/13, 14/32/15/33, 23/31/24) sont reliées entre elles par liaison physique.

9. Dispositif selon l'une des revendications 1 à 8, dans lequel les chaînes aliphatiques (11-15, 21-24, 31-33) comprennent au moins six atomes de carbone.

10. Dispositif selon l'une des revendications 1 à 9, dans lequel le polysaccharide est un alginate.

11. Procédé de fabrication du dispositif de traitement de plaie de la revendication 1, au cours duquel, on prend des macromolécules de polysaccharide (41) et des chaînes aliphatiques (44) pourvues chacune d'un unique groupe de fixation et on met en contact lesdites macromolécules de polysaccharide (41) et lesdites chaînes aliphatiques (44) afin de fixer des chaînes aliphatiques (44) sur les macromolécules (41).

12. Procédé selon la revendication 11, dans lequel on active le groupe de fixation des chaînes aliphatiques par ionisation.

13. Procédé selon la revendication 12, dans lequel les chaînes aliphatiques pourvues d'un groupe de fixation sont des molécules d'amine aliphatique.

14. Procédé selon l'une des revendications 11 à 13, dans lequel on met les molécules de polysaccharide et les chaînes aliphatiques pourvues d'un groupe de fixation dans une solution hydroalcoolique.

15. Dispositif naturel de traitement de plaie selon la revendication 1, dans lequel des molécules (60) d'un principe actif sont piégées dans des alvéoles (54, 55) du dispositif à l'état de gel.

16. Dispositif naturel de traitement de plaie selon la revendication 1, dans lequel des cellules vivantes (70) sont piégées dans des alvéoles (71, 72) du dispositif à l'état de gel.

## Claims

1. Natural device for treating wounds, arranged to change state in a reversible manner, by transition from the gel state to the solution state and vice-versa, comprising macromolecules ofpolysaccharide (10, 20,30) and aliphatic chains (11-15, 21-24, 31-33) fixed onto a single macromolecule of polysaccharide.

2. Device as claimed in Claim 1, wherein the aliphatic chains (11-15, 21-24, 31-33) are fixed by means of a chemical bond.

3. Device as claimed in Claim 2, wherein said chemical bond is an ionic bond.

4. Device as claimed in Claim 2, wherein said chemical bond is a covalent bond.

5. Device as claimed in Claim 4, wherein the chemical bond is an ester bond.

6. Device as claimed in any one of Claims 1 to 5, wherein each aliphatic chain is fixed by means of a fixing group.

7. Device as claimed in any one of Claims 1 to 6, wherein the aliphatic chains (21-24) fixed onto a macromolecule of polysaccharide (20) are, in the gel state, associated with aliphatic chains (11, 12/13, 31) fixed onto at least one other macromolecule of polysaccharide (10, 30).

8. Device as claimed in Claim 7, wherein the associated aliphatic chains (11/21, 12/22/13, 14/32/15/33, 23/31/24) are connected to each other by means of a physical bond.

9. Device as claimed in any one of Claims 1 to 8, wherein the aliphatic chains (11-15, 21-24, 31-33) comprise at least six carbon atoms.

10. Device as claimed in any one of Claims 1 to 9, wherein the polysaccharide is an alginate.

11. Method for producing the wound-treating device of Claim 1, during which macromolecules of polysaccharide (41) and aliphatic chains (44) each provided with a single fixing group, are taken and said macromolecules of polysaccharide (41) and said aliphatic chains (44) are brought into contact in order to fix aliphatic chains (44) onto the macromolecules (41).

12. Method as claimed in Claim 11, wherein the fixing group of the aliphatic chains is activated by ionisation.

13. Method as claimed in Claim 12, wherein the aliphatic chains provided with a fixing group are molecules of aliphatic amine.

14. Method as claimed in any one of Claims 11 to 13, wherein the molecules of polysaccharide and the aliphatic chains provided with a fixing group are placed in a water-alcohol solution.

15. Natural wound-treating device as claimed in Claim 1, wherein molecules (60) of an active principle are trapped in alveoles (54, 55) of the device in the gel state.

16. Natural wound-treating device as claimed in Claim 1, wherein living cells (70) are trapped in alveoles (71, 72) of the device in the gel state.

## Patentansprüche

1. Natürliches Wundbehandlungshilfsmittel, das für eine reversible Zustandsänderung ausgelegt ist, indem es vom Gelzustand in den Lösungszustand übergeht, das Polysaccharidmakromoleküle (10, 20, 30) und aliphatische Ketten (11-15, 21-24, 31-33) umfasst, die an ein einzelnes Polysaccharidmakromolekül fixiert sind.

2. Hilfsmittel nach Anspruch 1, wobei die aliphatischen Ketten (11-15, 21-24, 31-33) durch eine chemische Bindung fixiert sind.

3. Hilfsmittel nach Anspruch 2, wobei die chemische Bindung eine ionische Bindung ist.

4. Hilfsmittel nach Anspruch 2, wobei die chemische Bindung eine kovalente Bindung ist.

5. Hilfsmittel nach Anspruch 4, wobei die chemische Bindung eine Esterbindung ist.

6. Hilfsmittel nach einem der Ansprüche 1 bis 5, wobei jede aliphatische Kette mittels einer Fixierungsgruppe gebunden ist.

7. Hilfsmittel nach einem der Ansprüche 1 bis 6, wobei die an ein Polysaccharidmakromolekül fixierten aliphatischen Ketten (21-24) im Gelzustand mit aliphatischen Ketten (11, 12/13, 31) angeschlossen sind, die an mindestens ein weiteres Polysaccharidmakromolekül (10, 30) fixiert sind.

8. Hilfsmittel nach Anspruch 7, wobei die angeschlossenen aliphatischen Ketten (11/21, 12/22/13, 14/32/15/33, 23/31/24) untereinander durch eine physikalische Bindung gebunden sind.

9. Hilfsmittel nach einem der Ansprüche 1 bis 8, wobei die aliphatischen Ketten (11-15, 21-24, 31-33) mindestens sechs Kohlenstoffatome umfassen.

10. Hilfsmittel nach einem der Ansprüche 1 bis 9, wobei das Polysaccharid ein Alginat ist.

11. Herstellungsverfahren für eine Wundbehandlungshilfsmittel nach Anspruch 1, in dem Polysaccharidmakromoleküle (41) und aliphatische Ketten (44) verwendet werden, die jeweils mit einer einzigen Fixierungsgruppe versehen sind, und die Polysaccharidmakromoleküle (41) und die aliphatischen Ketten (44) in Kontakt gebracht werden, um die aliphatischen Ketten (44) an die Makromoleküle (41) zu fixieren.

12. Verfahren nach Anspruch 11, wobei die Fixierungsgruppe der aliphatischen Ketten durch Ionisierung aktiviert werden.

13. Verfahren nach Anspruch 12, wobei die aliphatischen Ketten, die mit einer Fixierungsgruppe versehen sind, aliphatische Aminmoleküle sind.

14. Verfahren nach einem der Ansprüche 11 bis 13, wobei die Polysaccharid moleküle und die aliphatischen Ketten, die mit einer fixierungsgruppe versehen sind, in eine Wasser-Alkohollösung eingebracht werden.

15. Natürliches Wundbehandlungshilfsmittel nach Anspruch 1, wobei Moleküle (60) eines Wirkstoffs in Kammern (54, 55) des im Gelzustand befindlichen Hilfsmittels eingeschlossen werden.

16. Natürliches Wundbehandlungshilfsmittel nach Anspruch 1, wobei Lebendzellen (70) in Kammern (71, 72) des im Gelzustand befindlichen Hilfsmittels eingeschlossen werden.
